# DEMANDE DE BREVET EUROPEEN

(11) **EP 1 878 402 A1**
(43) Date de publication de la demande: **16.01.2008**
(21) Numéro de dépôt: 06014538.0
(22) Date de dépôt: 13.07.2006
(51) Int. Cl.: A61C 1/18

(54) **Dispositif d'accouplement entre un arbre menant et un arbre mené d'une pièce à main à usage dentaire ou chirurgical**

(71) Demandeur: Bien-Air Holding SA, 2500 Bienne 6 (CH)
(72) Inventeur: Juan, Alain, 2072 Saint-Blaise (CH); Maitre, Luc, 2885 Epauvillers (CH); Bachmann, Robert, 2013 Colombier (CH)
(74) Mandataire: Ravenel, Thierry Gérard Louis

(57) **Abrégé**

L'invention concerne un dispositif d'accouplement entre un arbre menant (1A; 10A; 110A; 210A; 310A) et un arbre mené (1B; 10B; 110B; 210B; 310B) d'une pièce à main (12; 112) à usage dentaire ou chirurgical, ce dispositif comprenant un organe de liaison élastique (6; 60; 160; 260; 360; 460) qui relie l'arbre menant à l'arbre mené, caractérisé en ce que l'organe de liaison élastique comprend au moins une spire (6A; 60A; 160A; 260A) dont une section droite (S1; S2) est respectivement carrée ou rectangulaire.

L'invention concerne également un tel dispositif équipant une pièce à main articulée.

## Description

La présente invention concerne un dispositif d'accouplement entre un arbre menant et un arbre mené d'une pièce à main à usage dentaire ou chirurgical.

Les pièces à main du type contre-angle, c'est-à-dire des pièces à main dans lesquelles l'arbre menant ou arbre moteur et l'arbre mené, c'est-à-dire l'arbre qui est entraîné en rotation par l'arbre menant, forment un angle, sont d'un usage très répandu dans le domaine de la dentisterie ou encore de la chirurgie. Ces outils permettent en effet d'atteindre des endroits difficiles d'accès et offrent au praticien une bonne vision du champ opératoire.

La mise au point de telles pièces à main présente cependant un certain nombre de difficultés au rang desquels on peut citer le couplage en rotation entre l'arbre moteur et l'arbre mené.

Une première solution pour transmettre le couple de rotation entre deux arbres, respectivement menant et mené, qui forment entre eux un angle, autrement dit qui ne sont pas disposés dans le prolongement l'un de l'autre, consiste à utiliser un train d'engrenages. Un tel train d'engrenages présente toutefois un prix de revient élevé car il faut tailler des pignons en oblique et ce, en outre, avec une grande précision. Par ailleurs, en raison des vitesses de rotation élevées des arbres (des vitesses de l'ordre de 40'000 tours/minute sont couramment observées), de tels trains d'engrenages s'avèrent bruyants et ont tendance à s'user rapidement. Il faut donc prévoir une maintenance régulière de ces instruments, ce qui renchérit encore davantage leur prix de revient.

Une seconde solution pour coupler deux arbres formant entre eux un angle consiste à utiliser un ressort dont les spires d'extrémités sont fixées aux extrémités en regard des deux arbres. Une telle solution est plus simple à mettre en oeuvre et moins onéreuse. A l'usage, on s'est toutefois rendu compte que la fréquence élevée des cycles de compression/détente du ressort dus à la rotation des arbres favorisait les ruptures de fatigue d'un tel ressort. Ce problème est tout particulièrement sensible au démarrage de l'instrument, lorsque le ressort doit transmettre un couple élevé à l'arbre mené, et à l'arrêt, lorsque le ressort doit absorber l'inertie du système.

Pour résoudre ce problème, il a été proposé d'enfiler coaxialement deux ressorts l'un à l'intérieur de l'autre, le sens d'enroulement du premier ressort ou ressort intérieur étant contraire à celui du second ressort ou ressort extérieur. De la sorte, le déplacement des spires de l'un des ressorts, notamment au moment du démarrage et de l'arrêt du moteur de l'instrument, est compensé par le déplacement en sens contraire des spires de l'autre ressort. On parvient ainsi à réduire les contraintes de déformation s'exerçant sur les spires des ressorts et donc à diminuer les risques de rupture de fatigue. Toutefois, pour qu'un tel système à double ressort donne satisfaction, il faut que la surface extérieure du ressort intérieur soit en contact aussi étroit que possible avec la surface intérieure du ressort extérieur. Les ressorts doivent donc être boudinés avec grande précision, ce qui renchérit leur coût de fabrication.

La présente invention a pour but de remédier aux inconvénients susmentionnés ainsi qu'à d'autres encore en procurant un dispositif d'accouplement entre un arbre menant et un arbre mené d'une pièce à main à usage dentaire ou chirurgical qui soit notamment beaucoup plus résistant et fiable.

A cet effet, la présente invention concerne un dispositif d'accouplement entre un arbre menant et un arbre mené d'une pièce à main à usage dentaire ou chirurgical, ce dispositif comprenant un organe de liaison élastique qui relie l'arbre menant à l'arbre mené, caractérisé en ce que l'organe de liaison élastique comprend au moins une spire dont une section droite est carrée ou rectangulaire.

Grâce à ces caractéristiques, la présente invention procure un dispositif d'accouplement entre un arbre menant et un arbre mené dont la résistance aux ruptures de fatigue et donc la durée de vie sont considérablement améliorées. En effet, jusqu'à présent, la section droite des spires des organes de liaison élastiques utilisés pour coupler entre eux un arbre menant et un arbre mené était circulaire. Or, comme mentionné dans la partie introductive de la présente description consacrée à l'art antérieur, de tels organes de liaison ont tendance à casser rapidement en raison de ruptures de fatigue dues aux cycles à fréquence élevée de compression/détente auxquels ils sont soumis lorsque les arbres tournent à très haute vitesse.

Or, la Demanderesse a constaté qu'en utilisant un organe de liaison élastique dont la section droite des spires est carrée ou rectangulaire pour relier entre eux l'arbre menant et l'arbre mené d'une pièce à main, on améliorait considérablement la résistance aux ruptures de fatigue d'un tel ressort.

L'organe de liaison peut être obtenu par boudinage d'un fil de section carrée ou rectangulaire. Il peut également être obtenu par usinage d'un tube cylindrique monté sur une machine-outil et que l'on fait tourner sur lui-même autour de son axe de symétrie longitudinale tout en déplaçant un outil de coupe sur au moins une partie de la longueur du tube, parallèlement à l'axe de symétrie longitudinale du tube, de manière à usiner l'organe de liaison élastique dans ledit tube.

Selon une caractéristique complémentaire de l'invention, l'organe de liaison élastique est un ressort à spires.

Selon une autre caractéristique de l'invention, lorsque la section des spires du ressort est rectangulaire, le grand côté du rectangle qui s'étend selon le rayon des spires est au moins deux fois plus long que le petit côté du rectangle qui s'étend selon l'axe de symétrie longitudinale du ressort.

Dans le cas où le ressort présente une section rectangulaire conforme aux caractéristiques énoncées ci-dessus, on favorise la transmission du couple moteur du fait que la dimension radiale du ressort est grande, tandis que le relative minceur axiale dudit ressort confère à celui-ci une excellente souplesse selon son axe de symétrie longitudinale. On garantit ainsi une excellente transmission du couple, un fonctionnement souple et sans à-coup de même qu'une grande longévité du ressort.

Selon encore une autre caractéristique de l'invention, l'un au moins des arbres menant ou mené et l'organe élastique d'accouplement sont faits d'une seule pièce.

Grâce à ces caractéristiques, la présente invention procure un dispositif d'accouplement dans lequel l'organe élastique est déjà fixé à l'une au moins de ses extrémités à l'extrémité en regard de l'arbre menant ou de l'arbre mené, ce qui permet de réduire les coûts de fabrication. Par ailleurs, il est à noter que l'organe élastique venant de matière avec l'un au moins des deux arbres menant et/ou mené, le problème délicat de la fixation dudit organe élastique sur l'arbre concerné ne se pose plus, ce qui permet un montage beaucoup plus simple et plus fiable.

Selon encore une autre caractéristique de l'invention, l'organe de liaison élastique s'inscrit dans une enveloppe cylindrique dont le diamètre peut être différent ou égal à celui du ou des arbres avec lesquels il vient de matière.

Grâce à cette caractéristique, le constructeur dispose d'une liberté totale quant au choix du diamètre qu'il veut impartir à l'organe élastique d'accouplement. Il peut donc chercher le meilleur compromis possible entre la nécessaire souplesse de l'organe de liaison élastique pour garantir une transmission sans à-coup du couple moteur entre l'arbre menant et l'arbre mené, et l'indispensable rigidité de ce même organe de liaison permettant d'éviter les ruptures de fatigue. En particulier, plus le diamètre du ressort est petit, plus celui-ci est rigide et plus il risque de casser, notamment au niveau de ses attaches avec l'arbre avec lequel il vient de matière.

D'autres caractéristiques et avantages de la présente invention ressortiront plus clairement de la description détaillée qui suit de divers exemples de réalisation du dispositif d'accouplement selon l'invention, ces exemples étant donnés à titre purement illustratif et non limitatif seulement, en liaison avec le dessin annexé sur lequel:
- la figure 1 est une vue en perspective d'une portion de tube cylindrique creux de diamètre constant sur toute sa longueur dans lequel va être usiné le dispositif d'accouplement selon l'invention;
- la figure 2A est une vue du dispositif d'accouplement selon l'invention obtenu après usinage du tube représenté à la figure 1, ce dispositif comprenant un arbre menant et un arbre mené reliés entre eux par un organe de liaison élastique qui est fait d'une seule pièce avec les arbres menant et mené;
- la figure 2B est une vue analogue à celle de la figure 2A sur laquelle la portion d'arbre mené a été coupée;
- la figure 3A est une vue en perspective d'un tube cylindrique creux présentant localement une augmentation de son diamètre extérieur;
- la figure 3B est une vue du dispositif d'accouplement selon l'invention obtenu après usinage du tube représenté à la figure 3A, le diamètre extérieur des spires de l'organe de liaison élastique étant supérieur à celui des arbres menant et mené;
- la figure 4A est une vue en coupe d'une portion de tube cylindrique plein de diamètre constant sur toute sa longueur à l'intérieur duquel est pratiqué un alésage sur une longueur correspondant à celle de l'organe de liaison élastique;
- la figure 4B est une vue du dispositif d'accouplement selon l'invention obtenu après usinage du tube représenté à la figure 4A, ce dispositif comprenant un arbre menant plein et un organe de liaison élastique venant de matière avec l'arbre menant;
- la figure 4C est une vue en coupe d'un tube cylindrique plein présentant une première portion d'un premier diamètre extérieur et une seconde portion qui présente un second diamètre extérieur supérieur au premier, un alésage étant pratiqué dans la portion de tube de plus grand diamètre;
- la figure 4D est une vue du dispositif d'accouplement selon l'invention obtenu après usinage du tube représenté à la figure 4C;
- les figures 5A et 5B sont deux vues en coupe longitudinale de l'organe de liaison élastique selon l'invention, la section droite des spires étant respectivement carrée et rectangulaire, et
- la figure 6 est une vue en coupe longitudinale d'une pièce à main à usage dentaire ou chirurgical dans laquelle est intégré un dispositif d'accouplement selon l'invention, ce dispositif comprenant un arbre menant, un arbre mené et un organe de liaison élastique venant de matière avec les deux arbres, l'arbre mené étant creux;
- la figure 7 est une vue analogue à celle de la figure 6 sur laquelle l'arbre menant vient de matière avec l'organe de liaison élastique, ce dernier étant classiquement accouplé avec l'arbre mené;
- la figure 8 est une vue en coupe longitudinale d'une portion de barre dans laquelle est pratiqué un alésage dont le diamètre se réduit progressivement au fur et à mesure que l'on se rapproche de l'extrémité de l'arbre menant avec lequel l'organe de liaison élastique vient de matière, et
- la figure 9 est une vue schématique en coupe d'une pièce à main dentaire ou chirurgicale articulée équipée d'un dispositif d'accouplement selon l'invention.

La présente invention procède de l'idée générale inventive qui consiste à procurer un dispositif d'accouplement entre un arbre menant ou arbre moteur et un arbre mené comprenant un organe de liaison élastique entre l'arbre menant et l'arbre mené dont les spires sont de section carrée ou rectangulaire. Par rapport aux organes de liaison de l'art antérieur qui présentaient des spires de section circulaire, l'organe de liaison élastique selon l'invention dont la section des spires est carrée ou rectangulaire se révèle remarquablement bien adapté à la transmission d'un couple moteur, notamment en termes de résistance aux ruptures de fatigue. De plus, selon une caractéristique complémentaire de l'invention, il est prévu que l'organe de liaison élastique vienne de matière avec l'un au moins des deux arbres menant ou mené. L'organe de liaison est ainsi déjà fixé à l'une au moins de ses extrémités à l'extrémité en regard de l'arbre correspondant, ce qui permet de réduire les coûts. Surtout, le délicat problème de la fixation de l'organe de liaison élastique sur l'un et/ou l'autre des deux arbres menant et mené ne se pose plus, ce qui permet un montage beaucoup plus fiable.

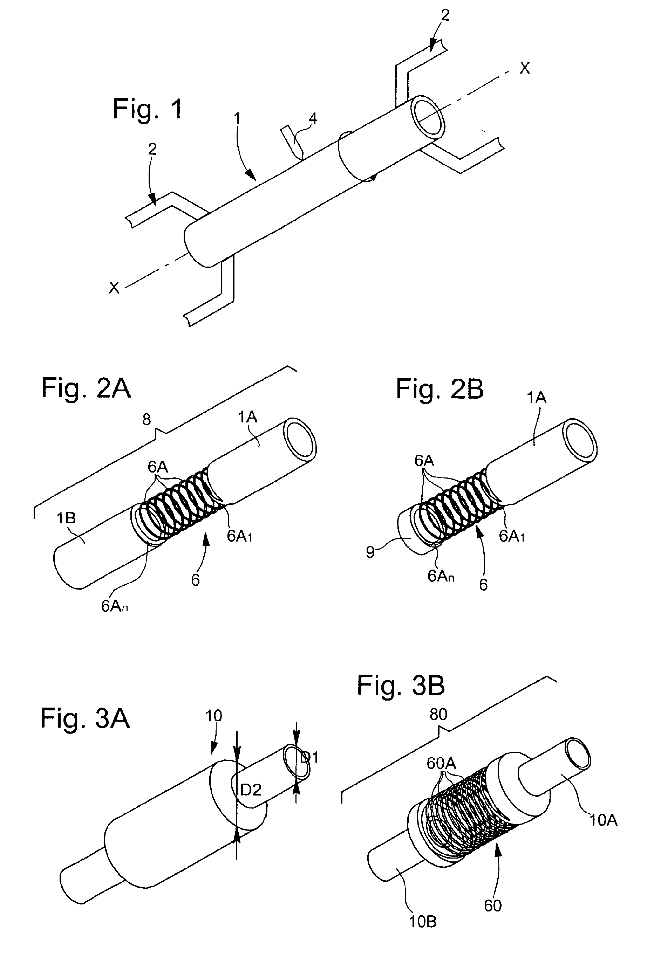

La figure 1 est une vue en perspective d'une portion de tube cylindrique 1 de diamètre constant sur toute sa longueur. Dans l'exemple représenté au dessin, ce tube 1 est creux. On verra ci-après que la présente invention peut également s'appliquer à un tube plein. Le tube 1 est maintenu à ses deux extrémités par deux jeux de pinces 2 comme on en trouve classiquement sur les machines-outils. Il est mis en rotation autour de son axe de symétrie longitudinale X-X tandis qu'on approche un outil de décolletage 4 tel qu'une fraise ou un taraud. L'outil 4 est approché suffisamment près du tube 1 pour pouvoir le percer à l'endroit de sa longueur où l'on souhaite que débute l'organe de liaison élastique 6 selon l'invention entre les deux arbres menant 1A et mené 1 B. Une fois que l'outil de décolletage 4 a percé le tube 1, on commande son avance parallèlement à l'axe de symétrie longitudinale X-X dudit tube 1. Sous l'effet conjugué de la rotation du tube 1 et de l'avance de l'outil 4, on matérialise une pluralité de spires 6A qui vont former l'organe de liaison élastique ou ressort 6. On comprendra aisément qu'en fonction de la vitesse d'avance de l'outil de décolletage 4 et de la vitesse de rotation du tube 1, on peut déterminer le nombre et la largeur des spires 6A du ressort 6. Finalement, on dégage l'outil de décolletage 4 du tube 1 lorsqu'on a obtenu la longueur voulue pour le ressort 6. Conformément à l'invention, on obtient ainsi un dispositif d'accouplement 8 comprenant un arbre menant 1 A et un arbre mené 1 B ainsi qu'un organe de liaison élastique sous la forme d'un ressort 6 qui est fait d'une pièce avec lesdits arbres 1A et 1 B (voir figure 2A). Un tel dispositif d'accouplement 8 présente un meilleur prix de revient qu'un dispositif conforme à l'art antérieur dans lequel les arbres menant/mené et l'organe de liaison élastique sont usinés séparément. Surtout, la présente invention permet de résoudre le délicat problème de la fixation de l'organe de liaison élastique sur les arbres menant/mené. Enfin, comme cet organe de liaison élastique est usiné à partir d'un tube ou d'une barre, le constructeur jouit d'une totale liberté quant au choix de la section des spires. Il n'est pas limité à une gamme de sections donnée.

Préférentiellement, comme il ressort de la figure 2A, la première spire 6A₁ qui relie le ressort 6 à l'arbre menant 1A sera taillée plus large que les spires suivantes de manière à former une attache fiable dudit ressort 6 audit arbre menant 1A. On pourra procéder de même avec la dernière spire 6Aₙ qui relie le ressort 6 à l'arbre mené 1 B.

Selon une variante de réalisation représentée à la figure 2B, on peut couper la portion d'arbre mené 1 B pour ne conserver que la portion d'arbre menant 1A prolongée par le ressort 6 et une portion annulaire terminale 9 qui facilitera le montage ultérieur du dispositif d'accouplement sur l'arbre mené. Il peut être également envisagé de couper la portion d'arbre menant 1A.

Une autre variante de réalisation est représentée aux figures 3A et 3B. Selon cette variante, on utilise un tube creux 10 de diamètre général extérieur D1 qui présente localement une augmentation de diamètre D2 (voir figure 3A). En procédant de manière analogue à celle décrite en liaison avec les figures 1 et 2A, 2B, on peut ainsi obtenir un dispositif d'accouplement 80 dont l'organe de liaison élastique 60 présente des spires 60A dont le diamètre extérieur est supérieur au diamètre extérieur des arbres menant 10A et mené 10B (voir figure 3B). Le constructeur dispose ainsi d'une liberté totale quant au choix du diamètre qu'il veut impartir à l'organe de liaison élastique. En particulier, il n'est pas limité dans ses calculs de dimensionnement par le diamètre extérieur des arbres menant 10A et mené 10B. Le constructeur peut donc chercher le meilleur compromis possible entre la nécessaire souplesse de l'organe de liaison élastique 60, garante d'un fonctionnement sans à-coup de la pièce à main, et l'indispensable rigidité de ce même organe d'accouplement permettant d'éviter les ruptures de fatigue. Bien qu'il puisse être envisagé d'usiner un organe de liaison dont le diamètre extérieur des spires soit inférieur à celui des arbres, on préfèrera tout de même le cas où ces spires sont plus grandes que les arbres. En effet, plus un ressort présente un faible diamètre, plus il est rigide et risque de casser.

On notera que, comme dans le cas illustré à la figure 2A, on peut éliminer la portion d'arbre mené 10B pour ne conserver que la portion d'arbre menant 10A prolongée par l'organe de liaison élastique 60. Il peut aussi être envisagé de couper la portion d'arbre menant 10A.

Jusqu'à présent, on s'est intéressé au cas où le dispositif d'accouplement selon l'invention était réalisé à partir d'une portion de tube creux. L'invention n'est toutefois nullement limitée à un tel choix et on peut très bien envisager d'utiliser une portion de tube plein comme on va maintenant le voir en liaison avec les figures annexées suivantes.

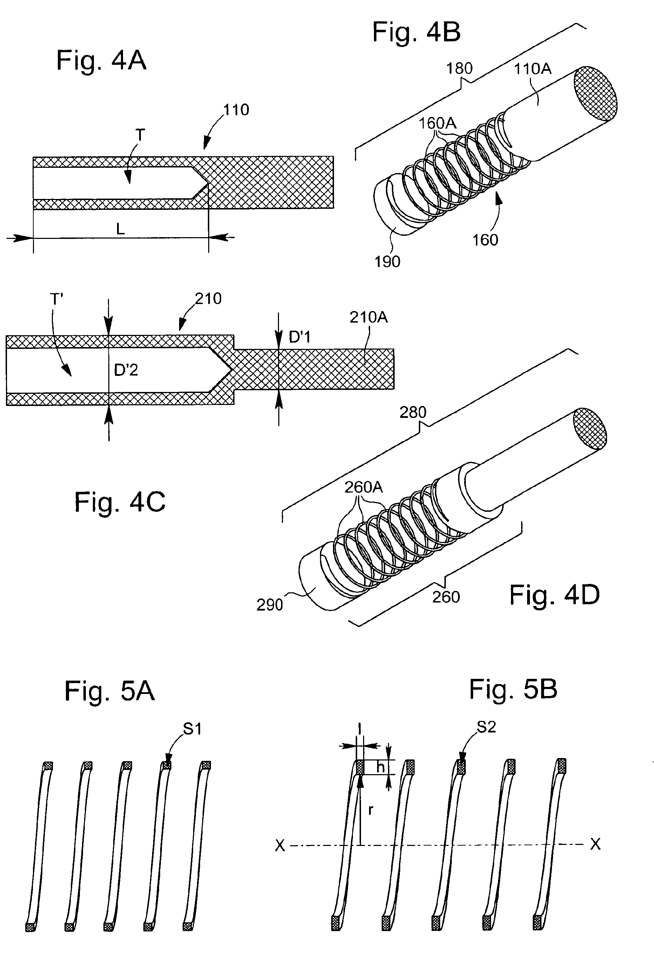

La figure 4A est une vue en coupe d'une portion de tube cylindrique 110 de diamètre constant sur toute sa longueur. Dans l'exemple représenté au dessin, ce tube 110 est plein. Pour permettre la mise en oeuvre de l'invention, il est donc nécessaire de pratiquer un alésage T sur une longueur L à l'intérieur du tube 110. L'alésage T est interrompu pour conserver une partie de tube plein qui constituera l'arbre menant 110A (ou l'arbre mené 110B). Après l'étape d'alésage intervient l'étape de décolletage qui, comme déjà décrit ci-dessus, permet d'usiner dans la partie alésée l'organe de liaison élastique 160 (voir figure 4B). Conformément à l'invention, on obtient ainsi un dispositif d'accouplement 180 comprenant un organe de liaison élastique 160 venant de matière avec l'arbre menant 110A (ou avec l'arbre mené 110B) qui est plein. Il suffira ensuite de raccorder l'extrémité libre 190 du ressort 160 à l'arbre mené (ou menant) en regard. On comprendra que le diamètre intérieur des spires 160A du ressort 160 est déterminé par le diamètre de l'alésage effectué.

Selon une variante représentée à la figure 4C, on peut également envisager d'utiliser un tube plein 210 qui présente une première portion 210A d'un premier diamètre extérieur D'₁ et une seconde portion qui présente un second diamètre extérieur D'₂ supérieur au premier. On effectue un alésage T' dans la portion de tube de plus grand diamètre D'₂ puis on usine l'organe de liaison élastique 260 par décolletage dans cette portion de tube. On peut ainsi réaliser (voir figure 4D) un dispositif d'accouplement 280 comprenant un organe de liaison élastique 260 dont le diamètre des spires 260A est totalement indépendant du diamètre de l'arbre menant (ou de l'arbre mené) et dont l'extrémité libre 290 peut être raccordée à l'extrémité libre en regard de l'arbre mené, respectivement de l'arbre menant.

Un autre avantage de la présente invention réside dans le fait que l'on peut, en sélectionnant convenablement le tube de départ, réaliser un organe de liaison élastique dont la section droite des spires est carrée ou rectangulaire, ce qui serait difficilement réalisable si l'on réalisait le ressort par les techniques classiques de boudinage. Ainsi, comme on peut le voir sur les figures 5A et 5B qui sont des vues en coupe longitudinale de l'organe de liaison élastique selon l'invention, la section S1 des spires est carrée (figure 5A) ou la section S2 des spires est rectangulaire (figure 5B). Dans ce dernier cas, le grand côté h du rectangle qui s'étend selon le rayon r des spires est préférentiellement au moins deux fois plus long que le petit côté l du rectangle qui s'étend selon l'axe de symétrie longitudinale X-X du ressort. Grâce à ces caractéristiques, on garantit une excellente transmission du couple, un fonctionnement souple et sans à-coup de même qu'une grande longévité du ressort.

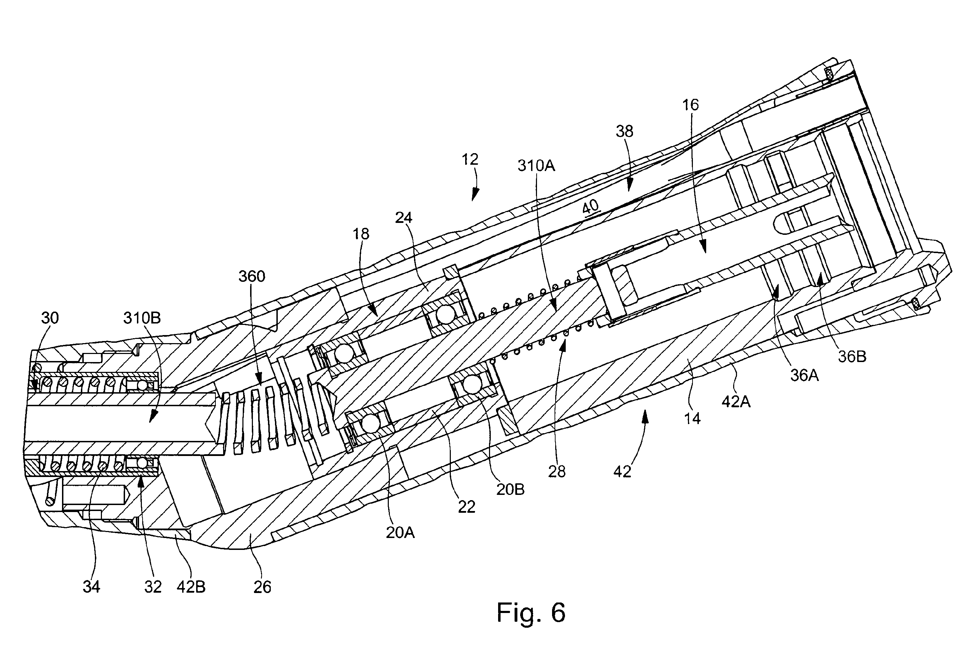

La figure 6 est une vue en coupe longitudinale d'une partie d'une pièce à main à usage dentaire ou chirurgical comprenant un dispositif d'accouplement conforme à la présente invention. Désignée dans son ensemble par la référence numérique 12, cette pièce à main encore appelée contre-angle comprend un bloc-support 14 pour un moteur électrique ou à air (non visible au dessin) à l'intérieur duquel est disposé de manière concentrique un dispositif 16 pour l'accouplement entre le moteur et l'arbre menant 310A. A son extrémité proximale, l'arbre menant ou arbre moteur 310A est porté par un palier 18 formé de deux roulements à billes annulaires 20A et 20B maintenus espacés par une entretoise 22. L'ensemble formé par les deux roulements à billes 20A et 20B et l'entretoise 22 est maintenu en place par une pièce porte-palier 24 qui est engagée sur une longueur limitée à l'intérieur d'une pièce 26 coudée selon un angle correspondant à celui du contre-angle 12. A son autre extrémité, la pièce porte-palier 18 est en appui contre le bloc-support 14. On voit au dessin que le contre-angle 12 comprend un ressort de rappel 28 enfilé sur l'arbre moteur 310A et qui exerce une contrainte sur les deux roulements à billes annulaires 20A et 20B. Ce ressort de rappel, par la force de poussée qu'il exerce, offre la possibilité d'un accouplement rapide en cas de désalignement entre l'arbre moteur 310A et le dispositif d'accouplement 16.

La pièce à main comprend également un arbre mené 310B disposé coaxialement à l'intérieur d'un alésage 30 pratiqué dans la pièce coudée 26. Cet arbre 310B est monté tournant à l'intérieur de la pièce coudée 26 via un roulement à billes annulaire 32 contraint par un ressort 34.

Des conduits 36A et 36B pour l'air et pour l'eau sont également prévus à l'extrémité distale de la pièce à main 12. Il est également prévu un passage longitudinal excentré 38 pour l'agencement d'une fibre optique 40 qui permet de guider la lumière jusqu'au champ opératoire.

L'ensemble des éléments constituant la pièce à main 12 décrits ci-dessus est logé à l'intérieur d'un corps 42 qui présente une partie avant 42A et une partie arrière 42B.

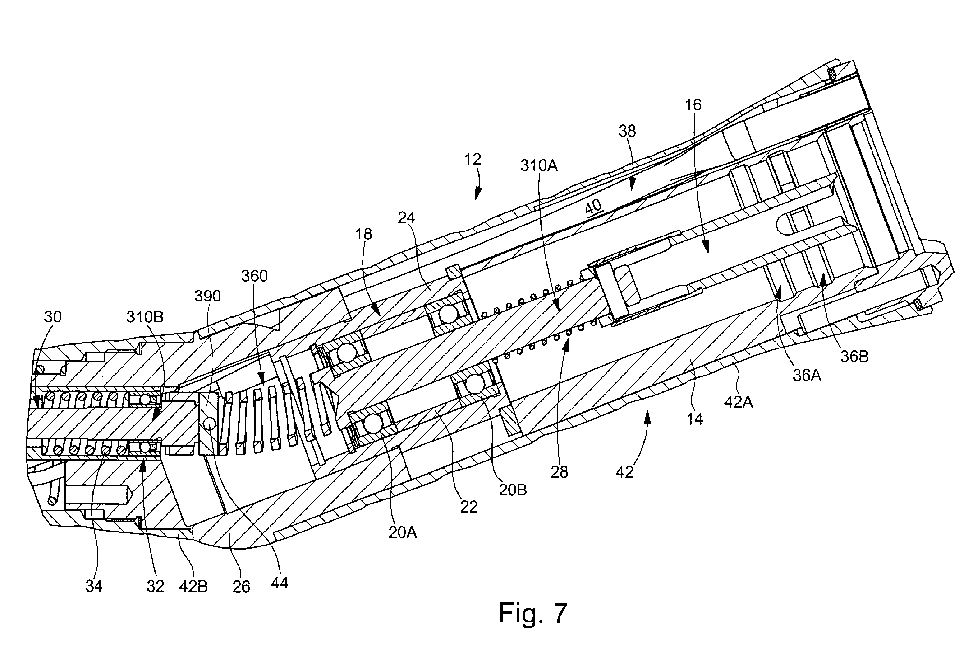

L'arbre menant 310A et l'arbre mené 310B sont reliés entre eux par un organe de liaison élastique 360. Conformément à la présente invention et comme il ressort de la figure 6, l'arbre menant 310A, l'arbre mené 310B et l'organe de liaison élastique 360 sont faits d'une seule pièce. Il va de soi que selon une variante illustrée à la figure 7, l'organe de liaison élastique 360 peut venir de matière avec l'arbre menant 310A seulement, et être fixé au niveau de son extrémité libre 390 par exemple au moyen d'une goupille 44 sur l'extrémité en regard de l'arbre mené 310B. L'inverse est également possible.

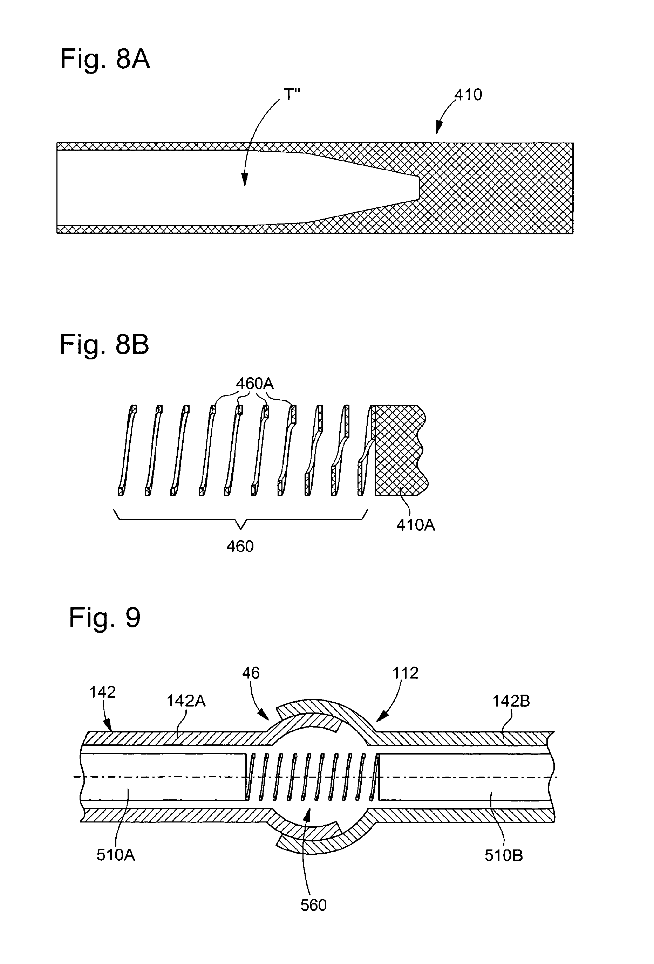

Il va de soi que la présente invention n'est pas limitée aux modes de réalisation qui viennent d'être décrits et que diverses modifications et variantes simples peuvent être envisagées par l'homme du métier sans sortir du cadre de l'invention tel que défini par les revendications annexées à la présente demande de brevet. En particulier, selon une variante illustrée à la figure 8, on réduit progressivement le diamètre de l'alésage T" pratiqué dans une barre 410 de façon à augmenter la section des spires 460A de l'organe de liaison élastique 460 au voisinage du point où celui-ci se raccorde avec la portion d'arbre menant et/ou mené avec laquelle il vient de matière. De la sorte, on déplace le point de fatigue depuis la région où les spires se raccordent à l'arbre vers le centre de l'organe de liaison élastique.

Par ailleurs, la présente invention s'avère particulièrement bien adaptée pour le cas où la pièce à main est articulée comme représenté à la figure 9. On voit en effet sur cette figure que les deux parties avant 142A et arrière 142B du corps 142 de la pièce à main 112 sont articulées à pivotement l'une par rapport à l'autre par exemple au moyen d'une rotule 46 disposée au même niveau que l'organe de liaison élastique 560. De la sorte, on profite pleinement de la présence de l'organe de liaison élastique selon la présente invention en procurant au praticien une pièce à main articulée dont il peut à volonté régler l'angle pour atteindre plus facilement des endroits à soigner difficiles d'accès.

## Revendications

1. Dispositif d'accouplement entre un arbre menant (1A; 10A; 110A; 210A; 310A; 410A; 510A) et un arbre mené (1B; 108; 310B; 510B) d'une pièce à main (12; 112) à usage dentaire ou chirurgical, ce dispositif comprenant un organe de liaison élastique (6; 60; 160; 260; 360; 460; 560) qui relie l'arbre menant à l'arbre mené, **caractérisé en ce que** l'organe de liaison élastique comprend au moins une spire (6A; 60A; 160A; 260A) dont une section droite (S1; S2) est respectivement carrée ou rectangulaire.

2. Dispositif d'accouplement selon la revendication 1, **caractérisé en ce que** l'organe de liaison élastique est un ressort à spires.

3. Dispositif d'accouplement selon l'une quelconque des revendications 1 ou 2, **caractérisé en ce que**, lorsque la section (S2) des spires est rectangulaire, un grand côté (h) du rectangle qui s'étend selon un rayon (r) des spires est au moins deux fois plus long qu'un petit côté (I) du rectangle qui s'étend selon un axe de symétrie longitudinale (X-X) de l'organe de liaison élastique.

4. Dispositif d'accouplement selon l'une quelconque des revendications 1 à 3, **caractérisé en ce qu'**au moins l'un des arbres menant ou mené et l'organe de liaison élastique sont faits d'une seule pièce.

5. Dispositif d'accouplement selon la revendication 4, **caractérisé en ce qu'**au moins la première, respectivement la dernière spire (6A₁; 6Aₙ) qui relie l'organe de liaison élastique à l'arbre menant, respectivement à l'arbre mené, présente selon l'axe de symétrie longitudinale dudit organe de liaison élastique une largeur supérieure à celle des spires suivantes, respectivement précédentes.

6. Dispositif d'accouplement selon l'une quelconque des revendications 4 ou 5, **caractérisé en ce que** la section des spires est de plus en plus grande au fur et à mesure que l'on se rapproche de l'extrémité en regard de l'arbre avec lequel l'organe de liaison élastique vient de matière.

7. Dispositif d'accouplement selon l'une quelconque des revendications 1 à 6, **caractérisé en ce que** le diamètre extérieur (D₂; D'₂) des spires de l'organe de liaison élastique est différent ou égal au diamètre extérieur (D₁; D'₁) de l'arbre menant et/ou de l'arbre mené.

8. Dispositif d'accouplement selon l'une quelconque des revendications 1 à 7, **caractérisé en ce que** le corps (142) de la pièce à main (112) est fait de deux parties avant (142A) et arrière (142B) articulées entre elles au moyen d'une articulation (46) prévue au niveau de l'organe de liaison élastique.
